(19) Europäisches Patentamt
European Patent Office
Office européen des brevets

(11) Veröffentlichungsnummer: **0 504 645 B1**

# (12) EUROPÄISCHE PATENTSCHRIFT

(45) Veröffentlichungstag der Patentschrift: **18.10.95**

(51) Int. Cl.[6]: **A61K 31/70**, C07H 11/00

(21) Anmeldenummer: **92103544.0**

(22) Anmeldetag: **02.03.92**

## (54) Verwendung von sulfatierten Oligosacchariden zur Behandlung arteriosklerotischer Erkrankungen und zur Verhinderung der Restenosierung.

(30) Priorität: **13.03.91 CH 765/91**

(43) Veröffentlichungstag der Anmeldung:
**23.09.92 Patentblatt 92/39**

(45) Bekanntmachung des Hinweises auf die Patenterteilung:
**18.10.95 Patentblatt 95/42**

(84) Benannte Vertragsstaaten:
**AT BE CH DE DK ES FR GB GR IT LI LU MC NL PT SE**

(56) Entgegenhaltungen:
**EP-A- 0 230 023**
**EP-A- 0 301 400**
**WO-A-88/06143**
**DE-A- 2 730 992**
**US-A- 4 840 940**

(73) Patentinhaber: **F. HOFFMANN-LA ROCHE AG**
**Postfach 3255**
**CH-4002 Basel (CH)**

(72) Erfinder: **Hosang, Markus**
**In den Reben 10**
**CH-4123 Allschwil (CH)**
Erfinder: **Iberg, Niggi**
**Schäublinstrasse 105**
**CH-4059 Basel (CH)**
Erfinder: **Tschopp, Thomas Beat**
**Juraweg 2**
**CH-4107 Ettingen (CH)**
Erfinder: **Wessel, Hans Peter**
**Im Bachacker 7**
**W-7843 Heitersheim (DE)**

(74) Vertreter: **Grossner, Lutz, Dr. et al**
**Grenzacher Strasse 124**
**Postfach 3255**
**CH-4002 Basel (CH)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung der Verbindungen der Formel (I)

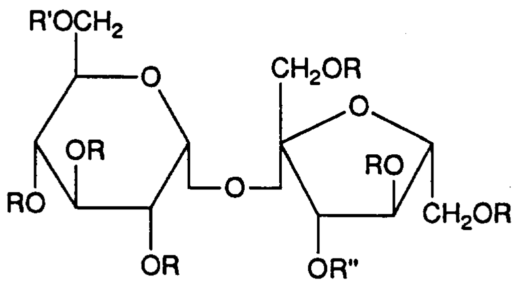

(I)

worin R Wasserstoff oder ein Rest $-SO_3M$; M ein Kation; R' und R" Wasserstoff oder ein α-glykosidisch verknüpfter sulfatierter Mono- oder Disaccharidrest ist;
wobei im Mittel mindestens eine $-SO_3M$-Gruppe pro Monosaccharideinheit anwesend ist,

als Wirkstoffe zur Herstellung von pharmazeutischen Präparaten zur Behandlung arteriosklerotischer Erkrankungen und zur Verhinderung der Restenosierung nach gefässchirurgischen Eingriffen und nach Organtransplantationen.

Als Kation M kommen alle physiologisch verträglichen Kationen in Betracht, z.B. Alkalimetallkationen wie $Na^+$ und $K^+$; Ammoniumionen und substituierte Ammoniumionen, die sich von tertiären Aminen wie Triäthylamin, oder Pyridin oder Imidazol ableiten; oder quaternäre Ammoniumionen wie Dodecyltrimethylammonium, Aethylpyridinium und Benzethonium; sowie Erdalkalimetallkationen wie $Ca^{++}$. Bevorzugt sind Verbindungen, in denen M $Na^+$ ist.

Als Sulfatierungsgrad wird die Anzahl von $-SO_3M$-Resten pro Monosaccharideinheit bezeichnet, die im Molekül im Mittel vorhanden sind. Der Sulfatierungsgrad 1 liegt somit z.B. dann vor, wenn ein Hexasaccharid der Formel I im Mittel 6 $-SO_3M$-Reste im Molekül enthält. Vorzugsweise beträgt der Sulfatierungsgrad in den Verbindungen der Formel I 2-3.

Die Verbindungen der Formel I können dadurch hergestellt werden, dass man ein entsprechendes Di-, Tri-, Tetra-, Penta- oder Hexasaccharid mit einem Sulfatierungsmittel behandelt und das Reaktionsprodukt in ein Salz überführt bzw. als solches isoliert. Beispiele solcher Saccharide sind Sucrose, Raffinose, Melezitose und Stachyose.

Die Sulfatierung dieser Saccharide kann mittels Methoden, die für die Sulfatierung von Hydroxygruppen an sich bekannt sind, vorgenommen werden.

Beispiele von Sulfatierungsmitteln, die für die Herstellung der Verbindungen der Formel I angewandt werden können, sind $SO_3$•Komplexe wie $SO_3$•Pyridin, $SO_3$•Trimethylamin, $SO_3$•Dioxan und $SO_3$•Dimethylformamid. Weitere Beispiele von Sulfatierungsmitteln sind Chlorsulfonsäure, Gemische von Chlorsulfonsäure und Schwefelsäure; und Piperidin-N-Sulfat.

Die Umsetzung erfolgt zweckmässig in einem geeigneten Lösungsmittel, insbesondere einem polaren Lösungsmittel, z.B. Dimethylformamid, Dimethylsulfoxid oder Hexamethylphosphortriamid. Die Reaktion kann bei Raumtemperatur oder erhöhter Temperatur, z.B. bei 20-70 °C durchgeführt werden, wobei durch Variation der Reaktionsdauer und -temperatur der Sulfatierungsgrad beeinflusst werden kann. Der jeweils erreichte Sulfatierungsgrad kann durch HPLC abgeschätzt werden. Die Aufarbeitung des Reaktionsgemisches bzw. die Isolierung des Reaktionsprodukts der Formel I kann nach an sich bekannten Methoden aus dem Reaktionsgemisch erfolgen, z.B. durch Gelfiltration oder Ultrafiltration.

Die als Ausgangsmaterialien eingesetzten freien Saccharide sind bekannt oder nach im Prinzip bekannten Methoden zugänglich. Für die Herstellung kommen enzymatische oder synthetische chemische Verfahren in Betracht. Die Oligosaccharide sind prinzipiell mit sequentiellen Synthesen oder Blocksynthesen aufbaubar. Hierbei werden glykosidische Bindungen durch Reaktion eines Glykosylakzeptors mit einem Glykosyldonor in Gegenwart eines geeigneten Katalysators hergestellt. Als Glykosyldonoren sind derivatisierte Glykosylverbindungen geeignet, die am anomeren Zentrum aktiviert sind, wie z.B. Chloride, Bromide, Fluoride, Acetate, Trichloracetimidate, Alkylthioderivate, usw.

Die Verwendung von Verbindungen der Formel I zur Verbesserung der Wundheilung sind aus EP-A-230023 bekannt. Eine anti-proliferative und Mucosa-protektive Wirkung von sulfatierten Oligosacchariden, die strukturell nicht nahe an den Verbindungen der Formel I liegen, ist aus EP-A-301400 bekannt.

Als Glykosylakzeptoren sind solche Saccharidderivate geeignet, bei denen die zu glykosidierenden OH-Gruppen frei und die übrigen vollständig oder teilweise geschützt sind. Bei nur teilweisem Schutz der übrigen OH-Gruppen kann die Glykosidierung selektiv erfolgen oder durch eine unterschiedliche Umgebung der Hydroxylgruppen in eine bestimmte Richtung gelenkt werden.

Die Verbindungen der Formel I hemmen die Migration und Proliferation von Zellen der glatten Muskulatur und verhüten proliferative arteriosklerotische Läsionen. Ihre blutgerinnungshemmende Aktivität ist geringer als diejenige von Heparin. Insbesondere haben die Verbindungen in vitro keine anticoagulante Aktivität, d.h. sie haben keinen oder nur sehr geringen Einfluss auf die Aktivität der Gerinnungsfaktoren Thrombin (F.IIa) und F.Xa. Die Verbindungen der Formel I können daher bei Erkrankungen eingesetzt werden, die nach Verletzungsreiz zur Proliferation glatter Muskelzellen führen. Insbesondere können die Verbindungen eingesetzt werden zur Verhinderung der Restenosierung nach gefässchirurgischen Eingriffen wie Endarteriektomie, Angioplastie, Bypass-Operationen mit Gefässtransplantaten und synthetischen Gefässprothesen sowie nach Organtransplantationen. Patienten mit progressiver Arteriosklerose können ebenfalls mit Verbindungen der Formel I behandelt werden.

Die blutgerinnungshemmende Wirkung wurde wie folgt ermittelt:

aPTT (activated partial thromboplastin time)-Test (vgl. Walenga et al., CRC Critical Reviews in Laboratory Sciences 22 (4) 361-389 (1986)): 100 µl zitriertes menschliches Plasma, das verschiedene Konzentrationen der Versuchsverbindung enthält, wird mit 100 µl Activated Thrombofax (Ortho Diagnostics, Raritan, N.J., U.S.A.) 8 Minuten bei 37 °C inkubiert. Dann werden 100 µl vorgewärmte 25 mM Calciumchloridlösung zugesetzt und die Gerinnungszeit wird im Fibrometer Coagulation Timer (Becton, Dickinson Basel) gemessen.

anti-Xa Clotting Assay: 25 µl zitriertes Plasma mit verschiedenen Konzentrationen der Versuchsverbindung werden mit 75 µl 1:100 verdünntem Faktor Xa (Diagnostic Reagents, Thame, Oxon, Grossbritannien) in Puffer (pH 7,3), der 0.63% Zitrat, 41 mM Imidazol, 82 mM NaCl und 0,1% Albumin enthält, gemischt. Nach 2 Minuten Erwärmen auf 37 °C werden 200 µl eines 1:1-Gemisches von Factor X Mangel-Plasma (Diagnostic Reagents) und Platelet Substitute (Diagnostic Reagents) zugesetzt und das Gemisch 20 Sekunden bei 37 °C inkubiert. Nach Zusatz von 100 µl vorgewärmter 50 mM Calciumchloridlösung wird die Gerinnungszeit im Fibrometer gemessen.

Die Aktivität der Versuchsverbindung wird als $IC_{50}$ angegeben, die jene Konzentration [µg/ml] angibt, welche zu einer Gerinnungszeit führt, die das Doppelte des Kontrollwerts beträgt.

Messung der Hemmung von Thrombin oder Faktor Xa mit Chromogenen Substraten (Teien et al., Thrombosis Research 10, 399-410 (1977)): Die Bestimmung erfolgte im Cobas-Bio Centrifugal Automatic Spectrophotometer. Die verwendete Pufferlösung bestand aus 50 mM Tris-Puffer, 180 mM NaCl, 7,5 mM EDTA-$Na_2$, 1% PEG 6000 und 0,02% Tween-80, pH 8,4. Die Testlösung bestand aus 50 ml Puffer, 30 µl Antithrombin III (1 U/ml, Kabi-Diagnostica) und 20 µl Plasma, das verschiedene Konzentrationen der Versuchsverbindungen enthielt. Im Analysenautomat wurden 30 µl Probenlösung und 20 µl Wasser mit 180 µl Thrombin (1 U/ml, Thrombin Reagent Roche Basel) in die Testküvette gegeben. Nach 240 Sekunden Inkubation bei 37 °C wurden 60 µl S-2238 (H-D-Phe-Pip-Arg-NH.pNA, Kabi Diagnostica, Möndal, Schweden, 0,75 mM in Wasser) und 20 µl Wasser zugesetzt. Die Freisetzung von pNA (p-Nitroanilin) wurde während 60 Sekunden bei 405 nm in 10-Sekunden-Intervallen im Vergleich zu Wasser als Blindwert verfolgt. Die Hemmwirkung wird als $IC_{50}$ angegeben, was die Konzentration [µg/ml] angibt, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2,8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

Die antiproliferative Aktivität der Substanzen wurde in Zellkulturen wie folgt ermittelt: Glatte Muskelzellen der Ratte (in DMEM mit 10% FCS bei 37 °C und 5% $CO_2$ kultiviert) wurden auf Zellkulturplatten in einer Dichte von $8x10^3$ Zellen/Vertiefung ausgebracht. Nach 4 Stunden wurde die Zahl der adherierten Zellen bestimmt und die zu testenden Substanzen (100 µg/ml) zugegeben. Als Vergleich dienten Zellen, denen nichts zugegeben wurde, und als positive Kontrolle diente Heparin (100 µg/ml). Die Zellen wurden für 7 Tage inkubiert und dann die Zellzahl bestimmt. Die antiproliferative Aktivität der einzelnen Substanzen wurde als % Inhibition im Vergleich zum nichtinhibierten Wachstum errechnet:

$$\% \text{ Inhibition} = \frac{\text{Zellzahl}_{(-)} - \text{Zellzahl}_{(inhib)}}{\text{Zellzahl}_{(-)} - \text{Zellzahl}_{(d=0)}} * 100$$

wobei

$\text{Zellzahl}_{(d=0)}$ = Zellzahl nach 4 h

$\text{Zellzahl}_{(-)}$ = Zellzahl der Zellen denen nichts zugegeben wurde, nach 7 Tagen

$\text{Zellzahl}_{(inhib)}$ = Zellzahl der Zellen mit 100 µg/ml der zu testenden Substanz

Die Resultate, die in den oben beschriebenen Versuchsanordnungen mit Verbindungen der Formel I erhalten wurden, sind in Tabelle 1 aufgeführt. Als Referenzverbindung diente Heparin.

Tabelle 1

| Verbindung von Beispiel | anti-proliferative Aktivität % Hemmung | Anti-Koagulationswirkung $IC_{50}$ [µg/ml] Gerinnungshemmung amidolytische Aktivität | | | |
|---|---|---|---|---|---|
| | | aPPT | Xa | Thrombin | F.Xa |
| 1 | 41 | 27 | >1000 | >1000 | >1000 |
| 2 | 25 | 150 | 250 | >1000 | >1000 |
| 3 | 49 | 11 | 32 | >1000 | >1000 |
| Heparin | 47 | 1,2 | 0,6 | 1,9 | 2,7 |

Die Versuchsresultate zeigen, dass die erfindungsgemässen Verbindungen eine antiproliferative Wirkung aufweisen, die derjenigen von Heparin entspricht oder nahekommt, im Gegensatz zu Heparin jedoch keine oder eine viel geringere anti-Koagulationswirkung zeigt.

Die Arzneimittel auf der Basis der erfindungsgemässen Verbindungen können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.

Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.

Beispiel 1

Eine Lösung von 2,0 g D-Raffinose-Pentahydrat in 50 ml absolutem Dimethylformamid wurde in Gegenwart von 10,3 g Schwefeltrioxid-Trimethylaminkomplex20 Stunden bei 60°C gerührt, wobei ein

Niederschlag ausfiel. Das Lösungsmittel wurde abdekantiert. Der Rückstand wurde mit Methanol gewaschen, in 64 ml wässriger 10%iger Natriumacetatlösung gelöst und eingeengt. Der Rückstand wurde mehrfach in Wasser aufgenommen und zur Entfernung von Trimethylamin eingedampft. Der Rückstand wurde zur Entfernung von Salzen gelchromatographiert (Sephadex® LH 20). Nach Gefriertrocknung wurden 5,28 g sulfatierte Raffinose erhalten, S = 19,89%, DS (durchschnittlicher Sulfatierungsgrad) etwa 2,8.

Beispiel 2

Eine Lösung von 2,0 g D-Melezitose-Monohydrat in 50 ml absolutem Dimethylformamid wurde in Gegenwart von 11,7 g Schwefeltrioxid-Trimethylaminkomplex18 Stunden bei 60 °C gerührt, wobei ein Niederschlag ausfiel. Aufarbeitung wie in Beispiel 1 ergab nach Zugabe von 69,1 ml 10%iger wässriger Natriumacetatlösung und Entsalzen 5,0 g sulfatierte Melezitose, S = 20,0%, DS ca. 3,0.

Beispiel 3

Eine Lösung von 850 mg Stachyosetetrahydrat in 20 ml absolutem Dimethylformamid wurde in Gegenwart von 4,5 g Schwefeltrioxid-Trimethylaminkomplex 24 Stunden bei 50 °C gerührt, wobei eine ölige Ausfällung entstand. Aufarbeitung wie in Beispiel 1 ergab nach Zugabe von 22 ml 10%iger wässriger Natriumacetatlösung und Entsalzen 2,0 g sulfatierte Stachyose, S = 19,5%, DS ca. 2,8.

Beispiel 4

| Tablette | | |
|---|---|---|
| 1 | Verbindung der Formel I | 500 mg |
| 2 | Laktose, wasserfrei | 150 mg |
| 3 | Mikrokristalline Cellulose | 150 mg |
| 4 | Polyvinylpyrrolidon | 40 mg |
| 5 | Talk | 50 mg |
| 6 | Magnesiumstearat | 10 mg |
| | Tablettengewicht | 900 mg |

Die Bestandteile 1-4 werden gesiebt und gemischt. Diese Mischung wird mit demineralisiertem Wasser granuliert und das getrocknete Granulat mit den Bestandteilen 5 und 6 vermischt. Man verpresst zu Tabletten geeigneter Form.

Beispiel 5

| Pellets: | | |
|---|---|---|
| 1 | Verbindung der Formel I | 500 mg |
| 2 | Mikrokristalline Cellulose | 200 mg |
| 3 | PRIMOJEL | 70 mg |
| 4 | Aromapulver | 10 mg |
| 5 | Talk | 20 mg |

Die gemischten und gesiebten Bestandteile 1-3 werden mit demineralisiertem Wasser ausreichend befeuchtet und mittels Extruder durch eine geeignete Lochscheibe gepresst. Das Extrudot wird auf einen Pelletierteller überführt, zu Kügelchen ausgerundet und anschliessend getrocknet. Man versetzt mit den gesiebten Bestandteilen 4 und 5 und füllt in Papiersachets (oder ähnliches) ab.

Beispiel 6

Injektionslösung:

Zur Herstellung einer Injektionslösung werden 50 mg Verbindung der Formel I sowie 0,5 mg Tris-Puffer in Wasser für Injektionszwecke ad 1 ml gelöst und der pH-Wert auf 7,4 eingestellt. Die Lösung wird sterilfiltriert und nach Abfüllen in Ampullen autoklaviert.

**Patentansprüche**

1. Verwendung von Verbindungen der Formel

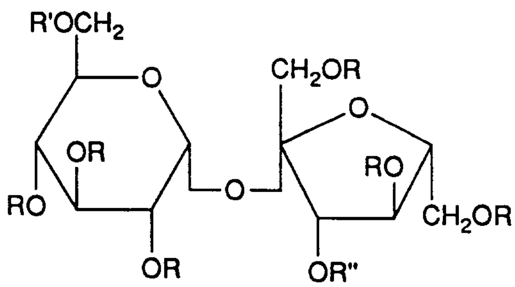

(I)

worin R Wasserstoff oder ein Rest $-SO_3M$; M ein Kation; R' und R'' Wasserstoff oder ein α-glykosidisch verknüpfter sulfatierter Mono- oder Disaccharidrest ist; und wobei im Mittel mindestens eine $-SO_3M$-Gruppe pro Monosaccharideinheit anwesend ist,
zur Herstellung von pharmazeutischen Präparaten zur Prävention und/oder Behandlung arteriosklerotischer Erkrankungen und zur Verhinderung der Restenosierung nach gefässchirurgischen Eingriffen und nach Organtransplantationen.

2. Verwendung gemäss Anspruch 1, wobei die Verbindung der Formel I ein sulfatiertes Tri- oder Tetrasaccharid ist.

3. Verwendung gemäss Anspruch 2, wobei die Verbindung der Formel I sulfatierte Raffinose ist.

4. Verwendung gemäss Anspruch 2, wobei die Verbindung der Formel I sulfatierte Melezitose ist.

5. Verwendung gemäss den Ansprüchen 1-4, wobei der Sulfatierungsgrad in den Verbindungen der Formel I etwa 2-3 beträgt.

**Claims**

1. Use of compounds of the formula

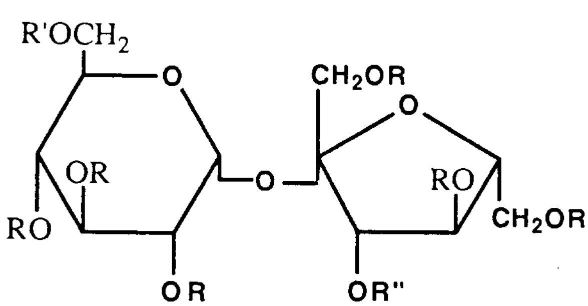

(I)

wherein R is hydrogen or a residue $-SO_3M$; M is a cation; R' and R'' are hydrogen or an α-glycosidically linked sulphated mono- or disaccharide residue; and at least one $-SO_3M$ group is present per monosaccharide unit,
for the manufacture of pharmaceutical preparations for the prevention and/or treatment of arteriosclerotic disorders and for the prevention of restenosis after invasive vascular surgery and after organ transplants.

2. Use in accordance with claim 1, wherein the compound of formula I is a sulphated tri- or tetrasaccharide.

3. Use in accordance with claim 2, wherein the compound of formula I is sulphated raffinose.

4. Use in accordance with claim 2, wherein the compound of formula I is sulphated melezitose.

5. Use in accordance with claims 1-4, wherein the degree of sulphation in the compounds of formula I is about 2-3.

## Revendications

1. Utilisation de composés de formule

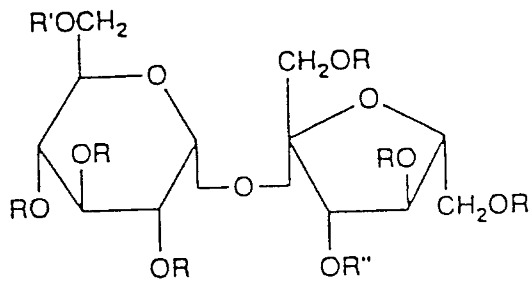

(I)

dans laquelle R est un atome d'hydrogène ou un radical $-SO_3M$; M est un cation, R' et R'' représentent un atome d'hydrogène ou un mono- ou disaccharide sulfaté relié par une liaison $\alpha$-glycosidique; et en moyenne au moins un groupe $-SO_3M$ étant présent par unité monosaccharidique,
pour la préparation de compositions pharmaceutiques destinées à la prévention et/ou au traitement de maladies artérioscléreuses et à empêcher la resténose après des interventions de chirurgie vasculaire et après des transplantations d'organes.

2. Utilisation selon la revendication 1, dans laquelle le composé de formule I est un tri- ou tétrasaccharide sulfaté.

3. Utilisation selon la revendication 2, dans laquelle le composé de formule I est le raffinose sulfaté.

4. Utilisation selon la revendication 2, dans laquelle le composé de formule I est le mélézitose sulfaté.

5. Utilisation selon les revendications 1 à 4, dans laquelle le degré de sulfatation dans les composés de formule I est environ 2-3.